(19) Europäisches Patentamt
European Patent Office
Office européen des brevets

(11) EP 3 530 269 A1

(12) **EUROPEAN PATENT APPLICATION**
published in accordance with Art. 153(4) EPC

(43) Date of publication:
**28.08.2019 Bulletin 2019/35**

(21) Application number: **17862278.3**

(22) Date of filing: **20.10.2017**

(51) Int Cl.:
***A61K 31/165*** (2006.01) ***A61K 9/14*** (2006.01)
***A61K 9/20*** (2006.01) ***A61K 9/48*** (2006.01)
***A61K 38/28*** (2006.01)

(86) International application number:
**PCT/KR2017/011636**

(87) International publication number:
**WO 2018/074879 (26.04.2018 Gazette 2018/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **21.10.2016 KR 20160137763**
**21.10.2016 KR 20160137764**

(71) Applicant: **Cellvertics Co., Ltd.**
**Seoul 06621 (KR)**

(72) Inventor: **SEO, Hong Seog**
**Seoul 04322 (KR)**

(74) Representative: **Nemec, Harald**
**Schwarz & Partner**
**Patentanwälte**
**Wipplingerstrasse 30**
**1010 Wien (AT)**

(54) **PHARMACEUTICAL COMPOSITION FOR PREVENTING OR TREATING DIABETES AND/OR HYPERLIPIDEMIA COMPRISING MIDORINE OR PHARMACEUTICALLY ACCEPTABLE SALT THEREOF AS ACTIVE INGREDIENT**

(57) The present disclosure relates to a pharmaceutical composition for preventing or treating diabetes and/or hyperlipidemia, which contains midodrine or a pharmaceutically acceptable salt thereof as an active ingredient, and to a use thereof.

FIG. 1

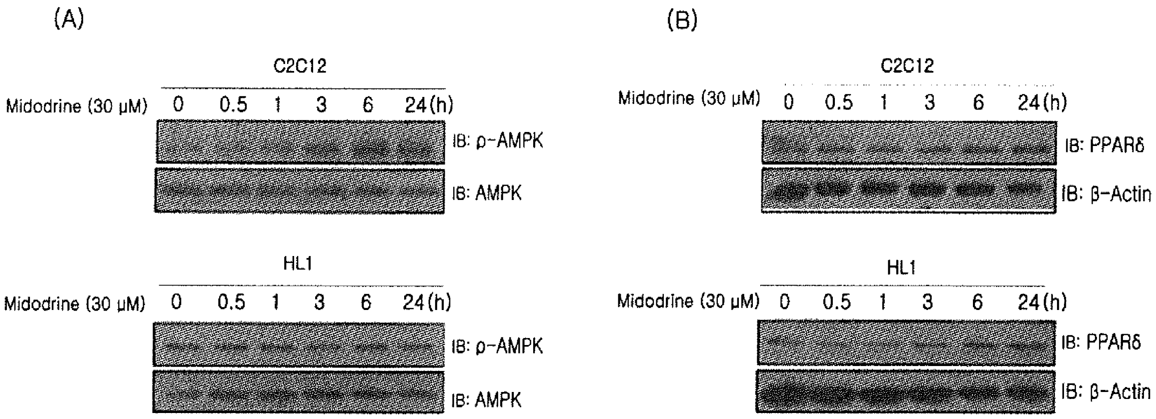


EP 3 530 269 A1

## Description

## BACKGROUND

### 1. Field of the Invention

The present disclosure relates to a pharmaceutical composition for preventing or treating diabetes and/or hyperlipidemia, which contains midodrine or a pharmaceutically acceptable salt thereof as an active ingredient.

### 2. Description of Related Art

Exercise training improves exercise tolerance by activating the remodeling program which cause phenotypic change in skeletal muscle. Adequate exercise is effective in improving pathological conditions such as metabolic diseases, heart diseases, etc.

In particular, AMPK (AMP-activated protein kinase), PPAR-δ (peroxisome proliferator activated receptor δ) and PGC-1α (peroxisome proliferator-activated receptor gamma coactivator 1α) are known as important factors involved in the phenotypic change in skeletal muscle for exercise tolerance.

AMPK is a heterotrimeric complex consisting of α/β/γ subunits. It is activated by phosphorylation by LKB1 and CaMKK ($Ca^{2+}$/calmodulin-dependent kinase kinase), which are upstream kinases of AMPK, during muscle contraction and exercise and is a major regulator of cell/organ metabolism related with glucose homeostasis, appetite and exercise physiology. PPAR-δ plays a key role in the transcriptional regulation of skeletal muscle metabolism. PGC-1α is a transcriptional coactivator which is involved in energy metabolism as a regulator of mitochondrial biosynthesis and function and is activated by endurance exercise of skeletal muscle.

AMPK is known to be able to target several transcription programs at the same time. These transcription programs are regulated by substrates such as PPAR-δ and PGC-1α and induce a genetic effect similar to that of exercise. In this regard, it has been reported that exercise mimetics targeting the AMPK/PPAR-δ signaling pathway can be a new pharmaceutical strategy of reprogramming muscles against resistance phenotypes (non-patent document 1).

In addition, some drugs that stimulate AMPK activation, such as A-769662, metformin, 5-aminoimidazole-4-carboxamide-1-β-D-ribofuranoside (AICAR) and resveratrol, have been reported to exhibit therapeutic effect for heart failure. The protective function of AMPK for heart disease can be achieved through important functions such as reduced production of reactive oxygen species in the cytoplasm, inhibition of the activity of angiotensin II, phosphorylation of cardiac troponin I, activation of PGC-1α, regulation of the expression of eNOS-NAD(P)H oxidase, regulation of estrogen-related receptors, regulation of energy balance and signal transduction in the heart, etc. As such, AMPK activation can improve the function of cardiac muscle directly or indirectly.

Because the AMPK activation (phosphorylation) and factors related thereto (PPAR-δ and PGC-1α) induce exercise-mimetic effects in skeletal muscle, such as glucose uptake, maintenance of energy metabolic homeostasis, improvement of cardiac function, etc., there have been consistent needs on drugs which activate AMPK as a target for diseases that can be treated with the exercise-mimetic effect.

(Non-patent document 1) Vihang A. Narkar et al., AMPK and PPARδ Agonists Are Exercise Mimetics, Cell, Volume 134, Issue 3, 8 August 2008, Pages 405-415.

## SUMMARY OF THE INVENTION

PROBLEMS TO BE SOLVED BY THE INVENTION

The inventors of the present disclosure have found out that diabetes and/or hyperlipidemia can be improved when the α1-adrenergic receptor (α1-AR) is activated with its agonist midodrine and have studied its mechanism of action consistently. As a result, they have identified that, when the α1-adrenergic receptor is activated, the expression of activated AMPK, PPAR-δ and PGC-1α is increased and, through this, exercise-mimetic effect can be induced in multiple organs such as skeletal muscle, cardiac muscle, liver, etc.

The present disclosure is directed to providing a pharmaceutical composition for treating or preventing diabetes, which contains midodrine or a pharmaceutically acceptable salt thereof as an active ingredient.

The present disclosure is also directed to providing a pharmaceutical composition for treating or preventing diabetes, which further contains insulin in addition to midodrine or a pharmaceutically acceptable salt thereof as the active ingredient.

The present disclosure is also directed to providing a method for treating diabetes using midodrine.

The present disclosure is also directed to providing a pharmaceutical composition for treating or preventing hyperlipidemia, which contains midodrine or a pharmaceutically acceptable salt thereof as an active ingredient.

The present disclosure is also directed to providing a method for treating hyperlipidemia using midodrine.
However, the technical problems to be solved by the present disclosure are not limited to those mentioned above and other problems not mentioned above will be clearly understood by those skilled in the art from the following description.

MEANS FOR SOLVING THE PROBLEM

In an aspect, the present disclosure provides a pharmaceutical composition for treating or preventing diabetes, which contains midodrine or a pharmaceutically acceptable salt thereof as an active ingredient.
The midodrine or the pharmaceutically acceptable salt thereof may induce AMPK activation.
The midodrine or the pharmaceutically acceptable salt thereof may induce the expression of PPAR-δ or PGC-1α.
The pharmaceutical composition may further contain insulin.
The pharmaceutical composition may contain 20-40 parts by weight of midodrine or a pharmaceutically acceptable salt thereof and 60-80 parts by weight of insulin based on the total weight of the pharmaceutical composition.
In another aspect, the present disclosure provides a method for treating diabetes, which includes a step of administering a pharmaceutically effective amount of midodrine or a pharmaceutically acceptable salt thereof to a subject.
In another aspect, the present disclosure provides a use of midodrine for preparation of a drug for treating or preventing diabetes.
In another aspect, the present disclosure provides a pharmaceutical composition for treating or preventing hyperlipidemia, which contains midodrine or a pharmaceutically acceptable salt thereof as an active ingredient.
The midodrine or the pharmaceutically acceptable salt thereof may induce AMPK activation.
The midodrine or the pharmaceutically acceptable salt thereof may induce the expression of PPAR-δ or PGC-1α.
In another aspect, the present disclosure provides a method for treating hyperlipidemia, which includes a step of administering a pharmaceutically effective amount of midodrine or a pharmaceutically acceptable salt thereof to a subject.
In another aspect, the present disclosure provides a use of midodrine for preparation of a drug for treating or preventing hyperlipidemia.
In the present disclosure, the α1-adrenergic receptor (α1-AR) agonist is not specially limited as long as it is a substance which acts on and activates the α1-adrenergic receptor. The adrenergic receptor has three types, α1, α2 and β. In the present disclosure, previously known compounds that activate the α1 type receptor may be used without limitation. Specifically, midodrine or a pharmaceutically acceptable salt thereof may be used.
Midodrine is marketed under the trade names of Amatine, ProAmatine, Gutron, etc. Its IUPAC name is (RS)-N-[2-(2,5-dimethoxyphenyl)-2-hydroxyethyl]glycinamide. It is represented by Chemical Formula I.

[Chemical Formula I]

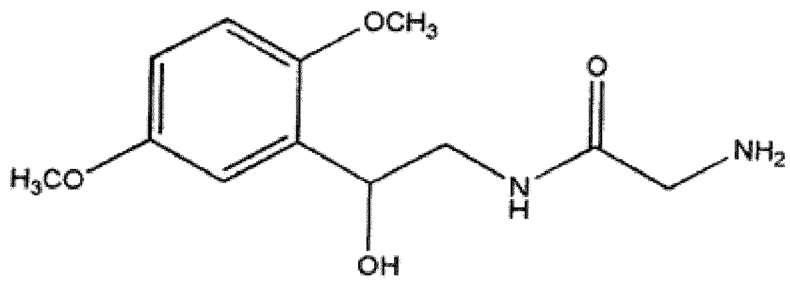

Midodrine is a prodrug which is converted to a desired compound after being administered into the body. After being administered into the body, it is changed into an active metabolite, desglymidodrine, which is capable of inducing an exercise-mimetic effect by activating the α1-adrenergic receptor and inducing AMPK activation and PPAR-δ or PGC-1α expression.
The compound represented by Chemical Formula 1 may form a "pharmaceutically acceptable salt". A suitable pharmaceutically acceptable salt is one commonly used in the technical field to which the present disclosure belongs, such as an acid addition salt, and is not specially limited. Specific examples of the pharmaceutically acceptable acid addition salt include inorganic acid salts such as hydrochloride, sulfate, nitrate, phosphate, perchlorate or bromate and organic acid salts such as acetate, methanesulfonate, ethanesulfonate, p-toluenesulfonate, fumarate, maleate, malonate,

phthalate, succinate, lactate, citrate, gluconate, tartrate, salicylate, malate, oxalate, benzoate, embonate, aspartate or glutamate. Examples of an organic base that may be used to prepare an organic base addition salt include tris(hydroxymethyl)methylamine, dicylohexylamine, etc. Examples of an amino acid that may be used to prepare an amino acid addition salt include natural amino acids such as alanine, glycine, etc.

The midodrine or the pharmaceutically acceptable salt thereof may induce AMPK activation.

AMPK serves as an energy sensor which senses and maintains the energy level in the body. For example, when the energy level in the cell is decreased due to metabolic stress or exercise, i.e., when the ratio of AMP/ATP is increased due to depletion of ATP, it is activated and accelerates the processes where ATP is consumed (e.g., fatty acid oxidation and glycolysis). AMPK activation induces metabolically important results in major target organs such as muscle. In particular, it is known to stimulate fatty acid oxidation and glucose uptake in skeletal muscle.

The midodrine or the pharmaceutically acceptable salt thereof may induce the expression of PPAR-δ or PGC-1α.

PPAR-δ is known to stimulate dissimilative energy metabolism in the cell by regulating AMPK and to play an essential role in the maintenance of balanced biological metabolism (homeostasis) such as anti-inflammatory action, etc.

PGC-1α is a major regulator of mitochondrial proliferation. Its expression is induced in response to severe metabolic changes such as exercise, starvation, coldness, etc. It is known to be regulated by AMPK, PPAR-δ, NAD-dependent deacetylase sirtuin-1 (SIRT1), etc.

The active ingredient according to the present disclosure can induce an exercise-mimetic effect and the exercise-mimetic effect may be an effect of preventing or treating a disease where AMPK activation is necessary.

In the present disclosure, the exercise-mimetic effect refers to the physiological effect exerted by exercise such as increased insulin sensitivity and oxidative phosphorylation of muscle, improvement in cardiac function (increased contractility), decreased in cholesterol, reduction of fat accumulation and body weight, etc. and is not specially limited.

In the present disclosure, the disease where AMPK activation is necessary refers to various diseases that may be caused by deactivation of AMPK without special limitation. For example, it may be a metabolic disease including diabetes and/or hyperlipidemia.

In the present disclosure, the "pharmaceutical composition" may further contain an existing active ingredient, an adjuvant, a pharmaceutically acceptable carrier, etc. The pharmaceutically acceptable carrier includes saline, sterile water, a Ringer's solution, buffered saline, dextrose solution, maltodextrin solution, glycerol, ethanol, etc.

The composition may be formulated into an oral formulation such as a powder, a granule, a tablet, a capsule, a suspension, an emulsion, a syrup, an aerosol, etc., a formulation for external application, a suppository or a sterile solution for injection.

In the present disclosure, it is obvious to those of ordinary skill that the range of "administration dosage" can be controlled variously depending on the body weight, age, sex and health condition of a patient, diet, the frequency and method of administration, excretion rate, the severity of a disease, etc.

In the present disclosure, the "subject" refers to a subject in need of treatment of a disease. More specifically, it means a mammal such as human, a non-human primate, mouse, rat, dog, cat, horse, cow, etc.

In the present disclosure, the "pharmaceutically effective amount" refers to an amount with which the maximum effect can be achieved with no side effect, which is determined in consideration of factors such as the disease to be treated and severity thereof, the age, sex and drug sensitivity of a patient, administration time, administration route, excretion rate, period of treatment and the drug(s) used together as well as other factors well known in the art. It can be easily determined by those skilled in the art.

The composition of the present disclosure is not limited in "administration method" as long as it can reach the target tissue. For example, it may be administered through oral administration, intraarterial Injection, intravenous Injection, transdermal Injection, intranasal administration, intratracheal instillation, intramuscular instillation, etc. A daily administration dosage may be about 0.0001-100 mg/kg. Specifically, a daily administration dosage of 0.001-10 mg/kg may be given once or several times a day.

In an example of the present disclosure, it was confirmed that coadministration of midodrine and insulin is effective in preventing or treating diabetes by increasing glucose uptake by cells.

In an example of the present disclosure, the effect of α1 -AR stimulation on the expression of exercise-mimetic genes in cardiac muscle cells and skeletal muscle cell was investigated. In addition, the effect on the heart, skeletal muscle and liver was compared *in vivo* using spontaneously hypertensive rat (SHR) which is an animal showing similar symptoms as metabolic syndrome in human.

As a result, it was found out that the stimulation of α1 -AR in the spontaneously hypertensive rat (SHR), which corresponds to human metabolic syndrome, by midodrine induces activation and increased expression of AMPK, PPAR-δ and PGC-1α in the heart, skeletal muscle and liver.

In addition, it was confirmed that the activation of PPAR-δ and PGC-1α by AMPK stimulation upon α1-AR stimulation is higher in the cardiac muscle than in the skeletal muscle or liver. These results are related with exercise-mimetic AMPK/PPAR-δ activation and suggest that they are independent of the muscle contraction effect by α1-AR stimulation. This is the first finding that pharmacologically stimulated cardiac muscle contraction contributes to the

activation of AMPK/PPAR-δ/PGC1α in the heart.

In the present disclosure, it was first found out through *in-vitro* and *in-vivo* experiments that α1-AR stimulation by midodrine activates AMPK/PPAR-δ/PGC-1α expression in the skeletal muscle irrelevantly of muscular motion. It has been already reported that the expression of these exercise-related genes is one of the adaptive responses for maintaining endurance exercise.

In the present disclosure, it was confirmed that α1-AR stimulation by midodrine induces AMPK/PPAR-δ/PGC-1a activation in the liver. In the present disclosure, it was confirmed that coadministration of midodrine and insulin leads to increased glucose uptake, suggesting that it is effective in preventing or treating diabetes.

Moreover, whereas the existing drugs for treating diabetes show side effects such as body weight increase and heart failure, midodrine exhibits body weight reducing, heart failure preventing and anti-inflammatory effects as well as the blood sugar lowering effect. Accordingly, it is very favorable for control of diabetes as compared to the existing diabetic drugs.

In the present disclosure, it was confirmed that the α1-AR agonist midodrine improves cardiac function by increasing the expression of PPAR-δ and PGC-1α, independently of the AMPK activation by α1-AR stimulation. It is because exercise training increases cardiac fractional shortening up to 40-50% and improves the rate of both contraction and relaxation.

Accordingly, it is thought that the α1-AR agonist improves exercise tolerance *in vivo* by improving cardiac function through direct simulation of cardiac contraction as well as indirect effect of exercise-mimetic AMPK/PPAR-δ activation by α1-AR stimulation in multiple organs including the heart, muscle and liver.

As such, it was first found out through the present disclosure that α1-AR stimulation by midodrine leads to cardiac exercise-mimetic effect through AMPK/PPAR-δ activation and, additionally, exhibits AMPK-independent cardiac movement effect by operating another exercise-mimetic program through PPAR-δ and PGC-1α expression.

In an example of the present disclosure, the effect of α1-AR stimulation by midodrine on the expression of exercise-mimetic genes in cardiac muscle cells and skeletal muscle cells was analyzed. In addition, the effect on the heart, skeletal muscle and liver was compared *in vivo* using spontaneously hypertensive rat (SHR) which is an animal showing similar symptoms as metabolic syndrome in human.

In addition, the effect of α1-AR stimulation on the function/size of the heart, adiponectin and fat levels, etc. was investigated.

As a result, it was found out that the stimulation of α1-AR in the spontaneously hypertensive rat (SHR), which corresponds to human metabolic syndrome, by midodrine induces activation and increased expression of AMPK, PPAR-δ and PGC-1α in the heart, skeletal muscle and liver and increases cardiac contractility without cardiac hypertrophy or further increase in blood pressure.

In addition, it was confirmed that the activation of PPAR-δ and PGC-1α by AMPK stimulation upon α1-AR stimulation by midodrine is higher in the cardiac muscle than in the skeletal muscle or liver. These results are related with exercise-mimetic AMPK/PPAR-δ activation and suggest that they are independent of the muscle contraction effect by α1-AR stimulation. This is the first finding that pharmacologically stimulated cardiac muscle contraction contributes to the activation of AMPK/PPAR-δ/PGC1α in the heart.

In addition, it was found out in the present disclosure that midodrine is more effective than other drugs inducing exercise-mimetic effect (e.g., atenolol). It is because midodrine also has an effect of improving cardiac contractility, whereas other drugs exert the exercise-mimetic effect only without direct effect on energy metabolism through cardiac muscle contraction. In addition, the α1-AR agonist is advantageous in that it exhibits the exercise-mimetic effect simultaneously in multiple organs alone, whereas other exercise mimetics are used together with other drugs.

In addition, it was found out in the present disclosure that, although the rats treated with midodrine showed increase in left ventricular ejection fraction comparable to that of the animals treated with atenolol, there was difference in the level of phosphorylated AMPK in the heart between the test groups. Although α-1 AR exhibits favorable effect on the heart, the degree of α-1 AR stimulation is important in terms of long-term prognosis. It is because highly reinforced cardiac α-1 AR drive results in pathological remodeling with contractile dysfunction, progressive fibrosis and reactivation of matricellular protein genes.

In addition, it was found out in the present disclosure that cardiac hypertrophy does not occur despite the α1-AR stimulation. Moreover, according to the present disclosure, left ventricular mass was smaller in the midodrine-treated group than in the atenolol-treated group. This can be explained by the difference in cardiac ATI expression.

In addition, it was first found out through *in-vitro* and *in-vivo* experiments that α1-AR stimulation activates AMPK/PPAR-δ/PGC-1α expression in the skeletal muscle irrelevantly of muscular motion. It has been already reported that the expression of these exercise-related genes is one of the adaptive responses for maintaining endurance exercise.

In addition, it was confirmed in the present disclosure that α1-AR stimulation by midodrine induces AMPK activation in the liver and the levels of total cholesterol, LDL-cholesterol and HDL-cholesterol decrease significantly. Therefore, it can be seen that it is effective for preventing or treating hyperlipidemia.

In addition, it was confirmed in the present disclosure α1-AR stimulation by midodrine reduces fat content in

adipocytes, suppresses fat synthesis/accumulation and reduces body weight and abdominal fat.

Moreover, because midodrine lowers cholesterol and prevents diabetes whereas the effect of the cholesterol-lowering drug statin on hyperlipidemia is accompanied by diabetes as a side effect, it can be used as a superior new therapeutic agent for hyperlipidemia with no side effect.

In another aspect, the present disclosure provides a dietary supplement for reducing blood sugar, which contains midodrine.

In another aspect, the present disclosure provides a dietary supplement for improving hyperlipidemia, which contains midodrine.

The dietary supplement of the present disclosure may contain, in addition to midodrine, various sub-ingredients and food additives. The dietary supplement may contain 0.1-90 wt% of midodrine based on the total weight of the dietary supplement.

For example, as the food additive, a sugar such as a monosaccharide, a disaccharide, a polysaccharide, a sugar alcohol, etc., a flavorant such as thaumatin, stevia extract, saccharin, aspartame, etc., a nutrient, a vitamin, an electrolyte, a sweetener, a colorant, an extender (e.g., cheese, chocolate, etc.), pectic acid, alginic acid, an organic acid, a protective colloidal thickener, a pH control agent, a stabilizer, an antiseptic, glycerin, an alcohol, a carbonating agent, etc. may be used.

The dietary supplement of the present disclosure can be prepared into various formulations without special limitation. Accordingly, it may be prepared into any formulation selected from a drink, a granule, a tablet, a powder, a pill and a capsule. The dietary supplement prepared into a drink, a granule, a tablet, a powder, a pill or a capsule is easy to carry and can be taken frequently anytime, anywhere.

As an example of the formulation of the dietary supplement of the present disclosure, a drink may consist of 0.1-40 wt% of midodrine and 60-99.9 wt% of purified water. Furthermore, additives such as taurine, citric acid, vitamin C, etc. may be added to the drink.

In addition, the drink may further contain various flavorants. As the flavorant, a natural flavorant such as thaumatin, stevia extract, etc. may be used.

Other formulations such as a granule, a tablet, a powder, a pill, a capsule, etc. may contain 1-50 wt% of midodrine and may further contain a gluing agent, a sweetener, a vitamin, a carbohydrate, etc.

## ADVANTAGE OF THE INVENTION

The composition of the present disclosure containing midodrine or a pharmaceutically acceptable salt thereof as an active ingredient may be usefully used to prevent and treat diabetes because it can increase glucose uptake into skeletal muscle cells and regulate metabolic disorder of mitochondria by increasing the expression of p-AMPK, PPAR-$\delta$ and PGC-1$\alpha$, which play a critical role in maintaining energy metabolism in the body.

In addition, the composition of the present disclosure containing midodrine or a pharmaceutically acceptable salt thereof as an active ingredient may be usefully used to prevent and treat hyperlipidemia because it can reduce fat content and lipid accumulation in adipocytes, reduce abdominal fat and body weight and regulate metabolic disorder of mitochondria.

Moreover, the composition of the present disclosure containing midodrine may be used as a dietary supplement for lowering blood sugar and/or improving hyperlipidemia.

## BRIEF DESCRIPTION OF THE DRAWINGS

FIG. 1 (A) and (B) show a western blot result showing that activation of the $\alpha$1-adrenergic receptor ($\alpha$1-AR) by midodrine increases the expression of p-AMPK (activated or phosphorylated AMPK) and PPAR-$\delta$ proteins in mouse skeletal muscle cells (C2C12) and mouse cardiac muscle cells (HL1).

FIG. 2A shows a result of investigating the expression of $\alpha$1-AR protein in the skeletal muscle of basal 4-week-old control rat (I), midodrine-administered rat (II), atenolol-administered rat (III) and unadministered 8-week-old control rat (IV) by western blotting, and FIGS. 2B-2E show a result of the expression of AMPK/PPAR-$\delta$/PGC1$\alpha$ proteins in the cardiac muscle, skeletal muscle, fat and liver of the above rat groups by western blotting.

FIG. 3A shows a result of measuring the enzymatic activity of SDH (succinate dehydrogenase) in the skeletal muscle of basal 4-week-old rat (I), midodrine-administered rat (II), atenolol-administered rat (III) and unadministered 8-week-old control rat (IV), and FIG. 3B shows a immunohistochemical staining result for cytochrome c oxidase in the skeletal muscle tissue of the above groups.

FIG. 4 shows a result of measuring the ATP level in the cardiac muscle, skeletal muscle and liver of basal 4-week-old rat (I), midodrine-administered rat (II), atenolol-administered rat (III) and unadministered 8-week-old control rat

(IV) by ELISA.

FIG. 5 shows the effect of midodrine on glucose uptake by insulin in mouse skeletal muscle cells (C2C12 cells).

FIG. 6 shows a result of measuring the heart rate of three groups of 4-week-old rat for 1 month.

FIG. 7 shows a result of investigating the expression level of HMG-CoA reductase protein (HMGCR) in the liver of rat groups.

FIG. 8A shows the effect of midodrine on the fat content in adipocytes, and FIG. 8B shows a result of investigating the effect of midodrine on the expression of PPAR-δ, p-AMPK and PGC-1α proteins which suppress fat synthesis/accumulation.

FIG. 9 shows a result of investigating the effect of midodrine on body weight (A) and abdominal fat weight (B).

## DETAILED DESCRIPTION OF THE INVENTION

Hereinafter, specific examples are presented to help understanding the present disclosure. However, the following examples are given only as examples of the present disclosure and it will be obvious to those of ordinary skill in the art that various changes and modifications can be made within the scope of the present disclosure. Also, it will be obvious that such changes and modifications belong to the scope of the appended claims.

### Example 1: Cell culturing and preparation of animal experiments

### 1-1: Cell culturing

Rat skeletal muscle cells (L6), mouse cardiac muscle cells (HL1) and mouse preadipocytes (3T3-L1) were placed onto a 6-well plate or a 24-well plate inside a 37 °C $CO_2$ incubator.

The L6 and HL1 cells were grown in a medium (DMEM) containing 10% FBS (fetal bovine serum) and 1% antibiotic until about 80% confluence, and then the medium was replaced with a medium containing 1% FBS for differentiation for 4 days. The drug was treated on day 3 of differentiation. All *in-vitro* experiments using the L6 and HL1 cells were conducted 24 hours after the drug treatment.

The 3T3-L1 cells were grown in a medium (DMEM) containing 10% FBS (fetal bovine serum) and 1% antibiotic until 100% and then cultured for 48 hours after replacing the medium with a fresh medium. After adding a differentiation medium and the drug, the cells were cultured for 48 hours. The composition of the differentiation medium was as follows: 0.0125 μmol/mL dexamethasone, 12.5 μmol/mL 3-isobutyl-1-methylxanthine, 10 μg/mL insulin, 10% FBS. After treating with the differentiation medium for 48 hours, the medium was replaced with an insulin medium containing 10 μg/mL insulin and 10% FBS and then treated for 48-96 hours. Then, after replacing the medium with a normal medium containing 10% FBS only, the cells were cultured for 24-48 hours and then used for experiments.

### 1-2: Animal experiments

Spontaneously hypertensive rat (SHR) is an experimental animal which is genetically hypersensitive. It is known to show hypertension which is the mostly similar to human primary hypertension. SHR begins to show increase in blood pressure at the age of usually 4-6 weeks and shows full hypertension at 8-12 weeks of age. Because SHR is frequently accompanied by hypertensive damage to target organs, such as cardiac hypertrophy, heart failure, kidney failure, etc., it is widely used in related researches as an animal model of primary hypertension, particularly hypertension with cardiac lesion.

3-week-old spontaneously hypertensive rats (SHRs) were kept under a standardized condition (21 °C, 41-62% humidity), with periodic light/dark (10/14 hours) cycles and free access to water and feed. All animal experiments were carried out to comply with the rules of Korea University Animal Science (KUIACUC-2012-100). The experimental procedures and conditions were approved by the Korea University Institutional Animal Care and Use Committee.

After 1 week of accommodation, the rats were divided into 4 groups (6 rats per group) as follows: group I (basal control, sacrificed at week 4), group II (administered with midodrine for 4 weeks), group III (administered with atenolol for 4 weeks), and group IV (control with no drug administration for 4 weeks). The group I and group IV were given normal oil-fat feed (K-H4 pellet, sniff) without any drug administration. The group II was given the same feed together with midodrine-containing drinking water (0.3 mg/kg/day). The group III was given the same feed together with atenolol-containing drinking water (1 mg/kg/day).

The rats of group I were euthanized at week 4, whereas the rats of other groups were euthanized at week 8 after drug administration for 4 weeks. Blood samples were taken from the inferior vena cava and the heart, aorta, liver, skeletal muscle and visceral fat (abdominal fat) were incised cleanly for use in experiments. The recovered organs were kept in a refrigerator at -80 °C or in 10% formalin for immersion fixation.

## Example 2: Experimental methods

### 2-1: ELISA for ATP measurement

0.02 g of liver tissue isolated from the rats of Example 1-2 was homogenized in 500 μL of PBS. The homogenate was centrifuged at 1500xg (or 5000 rpm) for 15 minutes and the supernatant was recovered. After adding 100 μL of standard materials or samples to adequate wells of a pre-coated microtiter plate, 10 μL of the remaining solution was added to the sample. After adding 50 μL of the conjugate to each well and then mixing, the plate was covered and incubation was conducted at 37 °C for 1 hour. After adding 50 μL of each of substrates A and B to each well, incubation was conducted at 37 °C for 15 minutes. After adding a stop solution to each well, optical density was measured at 450 nm using a microplate reader.

### 2-2: Measurement of enzymatic activity of SDH (succinate dehydrogenase)

After adding 10 μL of protease inhibitor cocktail per 1 mL of PBS (phosphate-buffered saline) to each well (500 μL), 410 μL of a culture solution prepared by mixing 1 M phosphate buffer (25 μL x 25 = 625 μL), 0.2 M sodium succinate (125 μL x 25 = 3125 μL), NBT (25 μL x 25 = 625 μL) and D.W. (235 μL x 25 = 5875 μL) per well was heated to 37 °C for 20 minutes. After adding 500 μL of the PBS prepared above to 0.02 g of the skeletal muscle tissue isolated from the rats of Example 1-2, the skeletal muscle tissue was crushed for a short time such that the temperature was not increased to avoid destruction of enzymes. After conducting centrifugation at 13000 rpm and 4 °C for 5 minutes, the supernatant was recovered and transferred to a fresh tube. After adding 410 μL of a culture solution to a tube, enzymatic reaction was conducted by adding 90 μL of the sample. After adding 410 μL of distilled water (DW) to another tube and adding 90 μL of the sample, the absorbance of the diluate was measured. After adding the enzymatic reaction tube and the another tube in water bath at 37 °C, respectively, reaction was conducted for 30 minutes. The reaction was terminated by putting the tube in ice. After adding 200 μL to each well of a 96-well plate, absorbance was measured at 550 nm. Then, the enzymatic activity of succinate dehydrogenase (SDH) was measured by the following equation.

Enzymatic activity = (absorbance of enzymatic reaction solution - absorbance of diluted enzymatic reaction solution) / protein content (Bradford 595 nm)

### 2-3: Western blot

After recovering proteins from cells using PREP™ protein extraction solution (iNtRON Biotechnology, Seongnam-si, Gyeonggi-do, Korea), 20-30 μg of the proteins were separated by loading onto a 10% SDS-PAGE (SDS-polyacrylamide gel electrophoresis) gel and then transferred to nitrocellulose paper (GE Healthcare, UK). After blocking the membrane in TBS (Tris-buffered saline) containing 5% (w/v) skim milk and 0.05% (v/v) Tween-20 at 4 °C overnight or at room temperature for 2 hours, primary antibodies for AMPKα (α subunit), phosphorylated AMPKα (phosphorylated at Thr172), PPAR-δ and PGC-1α (Cell Signaling Technology, Inc., Danvers, MA, USA) were added. After incubating at room temperature for 2 hours or at 4 °C overnight, reaction was conducted with horseradish peroxidase-conjugated anti-rabbit secondary antibodies (Santa Cruz Biotechnology, Santa Cruz, CA, USA). The Clarity Western ECL Substrate kit (Bio-Rad, Hercules, CA, USA) was used as a detection reagent. Images were obtained manually using the Kodak GBX developer and fixer (Kodak, Rochester, NY, USA). X-ray fills were purchased from Agfa (Mortsel, Belgium). The contents of the proteins derived from the cells were measured by the Bradford method.

### 2-4: Measurement of glucose uptake in cells

After treating the mouse skeletal muscle cells (C2C12 cells) cultured according to Example 1-1 respectively with 100 μM/L insulin, 30 μM/L midodrine or a mixture of 30 μM/L midodrine and 100 μM/L insulin (1:2 (w/w)), the level of glucose uptake was measured with 2-deoxyglucose.

### 2-5: Measurement of cardiac function and body weight

After anesthetizing the 8-week-old rats of Example 1-2 by intramuscular injection of zoletil (8 mg/kg) and xylzine (2 mg/kg), the rats were laid on their left sides and M-mode echo images were obtained. All the examination was conducted using Vivid 7 (GE Medical Systems, Milwaukee, WI, USA) equipped with a 12 MHz transducer. After acquiring optimum 2D uniaxial images for the left ventricle in the level of papillary muscles, M-mode tracing and electrocardiographic

recording were conducted at the same time at a rate of 100 mm/s. >From at least three consecutive cardiac cycles for the M-mode tracing, the heart wall thickness, left ventricular ejection fraction (measure of contractility) and left ventricular mass were measured by echocardiography (American Society for Echocardiography).

Body weight was measured for the same rats whose cardiac function was measured.

In addition, the heart rate of the 4-week-old rats of Example 1-2 was measured over 1 month.

**2-6: Measurement of blood biochemical properties and adiponectin**

The total cholesterol, HDL (high-density lipoprotein) cholesterol, LDL (low-density lipoprotein) cholesterol and triglyceride levels of the blood of the rats of Example 1-2 were measured by chromogenic enzyme assay (Roche Diagnostics GmbH; Mannheim, Germany).

The adiponectin level in the blood of the rats of Example 1-2 was measured using a rat adiponectin detection ELISA kit (Abcam, Cambridge, UK). The measured adiponectin level was normalized to the visceral fat weight (g).

**2-7: Confirmation of suppression of fat content in adipocytes**

After differentiating the mouse-derived preadipocytes (3T3-L1) cultured according to the method of Example 1-1 into adipocytes and then treating with 30 μM/L midodrine or a mixture of 30 μM/L midodrine and 50 μM/L GSK0660 (PPAR (peroxisome proliferator-activated receptor) β/δ inverse agonist (1:2 (w/w), the fat content in the adipocytes was observed visually using a microscope (OLYMPUS IX71).

**2-8: Statistical analysis**

Continuous variables were recorded as mean ± standard deviation (SD). The difference of the variables throughout the 4 groups was analyzed by the Kruskal-Wallis test. The difference between two groups was evaluated by the Mann-Whitney U-test. P-values < 0.05 were considered as statistically significant. All the statistical analysis was conducted using SPSS (ver. 20.0; SPSS Inc., Chicago, IL, USA).

**Example** 3: **Experimental results**

**3-1: Effect of administration of midodrine to skeletal muscle cells or cardiac muscle cells on AMPK phosphorylation *in vitro***

In order to investigate the effect of α1-AR stimulation on AMPK and AMPK phosphorylation (activation) *in vitro,* the mouse skeletal muscle cells (C2C12) and the mouse cardiac muscle cells (HL1) cultured in Example 1-1 were treated with 30 μM of midodrine, which is an α1-AR agonist, and then western blot was conducted as described in Example 2-3.

As a result, AMPK was expressed by α1-AR stimulation in the skeletal muscle cells and the expression of phosphorylated AMPK was increased with the concentration of midodrine, as shown in FIG. 1 (A) and (B). Accordingly, it was confirmed that α1-AR stimulation is related with AMPK activation. In addition, the α1-AR stimulation by the administration of midodrine also resulted in the expression and phosphorylation of AMPK in the cardiac muscle cells as well.

Because the effect of midodrine in the skeletal muscle (AMPK activation) is also observed in the cardiac muscle simultaneously with the previously known improvement in cardiac muscle contractility(AMPK activation and cardiac muscle contraction increase), it was confirmed that the α1-AR stimulation provides an additional effect of cardiac muscle motion. That is to say, although only the possibility of contractility increase of cardiac muscle cells was known through *in-vitro* experiments thus far, the result of this example suggests an exercise-mimetic effect in skeletal muscle and cardiac muscle cells due to the increase and phosphorylation of AMPK.

**3-2: Expression of AMPK, PPAR-δ and PGC-1α proteins in cardiac muscle, skeletal muscle, fat and liver**

In order to investigate the presence of α1-AR in skeletal muscle, western blotting was conducted for basal 4-week-old control rat (I), midodrine-administered rat (II), atenolol-administered rat (III) and unadministered 8-week-old control rat (IV) according to the method of Example 2-3. As shown in FIG. 2A, the group IV showed the highest expression level of α1-AR.

In addition, the expression level of AMPK, PPAR-δ and PGC-1α proteins in the cardiac muscle, skeletal muscle, fat and liver derived from the rats of the four groups was investigated by conducting western blotting according to the method of Example 2-3. As seen from FIG. 2B (cardiac muscle), FIG. 2C (skeletal muscle), FIG. 2D (fat) and FIG. 2E (liver), for the cardiac muscle, the expression of the phosphorylated AMPK protein was higher for the group I than the

group IV ($p < 0.05$). The midodrine-administered group II showed remarkably higher AMPK expression in the cardiac muscle, skeletal muscle and fat as compared to the control group IV. The atenolol-administered group III showed no higher expression of the AMPK protein when compared with the control group IV of the same age.

Notably, whereas the cardiac muscle showed much higher increase in PGC-1$\alpha$ and PPAR-$\delta$ than that of AMPK, the skeletal muscle showed much lower increase in PGC-1$\alpha$ and PPAR-$\delta$ than that of AMPK. This means that, whereas the exercise-mimetic effect of cardiac muscle increases the expression of PGC-1$\alpha$ and PPAR-$\delta$ directly together with AMPK activation, the skeletal muscle does not show increase in PGC-1$\alpha$ and PPAR-$\delta$ relative to AMPK.

**3-3: Change in expression of mitochondrial oxidase in skeletal muscle**

The enzymatic activity of SDH, which is an enzyme of the mitochondrial oxidation process (TCA cycle), was measured in skeletal muscle according to the method of Example 2-2. As seen from FIG. 3A, the midodrine-administered group II showed the highest increase.

In addition, when immunohistochemical staining was conducted on skeletal muscle tissue for cytochrome c oxidase, which is a mitochondrial oxidase, the midodrine-administered group II showed the highest increase as can be seen from FIG. 3B.

From these results, it can be seen that midodrine enhances metabolic action.

**3-4: Comparison of ATP level in tissue**

The ATP level in the heart, skeletal muscle and liver of basal 4-week-old rat (I), midodrine-administered rat (II), atenolol-administered rat (III) and unadministered 8-week-old control rat (IV) was measured by ELISA according to the method of Example 2-1.

As seen from FIG. 4, the heart tissue of the midodrine- or atenolol-administered group showed higher ATP level despite higher contractility as compared to control SHRs.

**3-5: Effect on glucose uptake in cells**

The effect of midodrine on glucose uptake by insulin in mouse skeletal muscle cells (C2C12 cells) was investigated. As seen from FIG. 5, glucose uptake was increased remarkably when a mixture of insulin and midodrine was treated as compared to treatment with insulin alone.

From this result, it can be seen that midodrine is effective in treating diabetes.

**3-6: Effect of midodrine administration on cardiac function and body weight in *in-vivo* animal model**

If midodrine administration increases AMPK, PPAR-$\delta$ (peroxisome proliferator activated receptor-$\delta$) and PGC-1$\alpha$ (peroxisome proliferator-activated receptor gamma coactivator-1$\alpha$) also in cardiac muscle *in vivo,* it can be seen that the AMPK/PPAR-$\delta$/PGC1$\alpha$ coexpression cascade with its exercise-mimetic effect proven for the skeletal muscle can improve the cardiac muscle contractility. Therefore, cardiac function and body weight were investigated *in vivo* according to the method of Example 2-5.

As seen from the echocardiographic data for 8-week-old rats presented in Table 1, the midodrine-administered rat group II and the atenolol-administered rat group III showed higher left ventricular performance than the unadministered control group IV.

The left ventricular mass was the lowest for the group II, but no significant difference was observed between the groups. The group III showed the highest body weight.

In addition, when measuring the heart rate of the three groups (blue: control group, red: midodrine-administered group, green: atenolol-administered group) of 4-week-old rats over 1 month, the atenolol group showed the lowest heart rate at the end of the experiment (FIG. 6).

[Table 1]

| Parameters | Midodrine (group II) | Atenolol (group III) | Control (group IV) | P-value |
|---|---|---|---|---|
| Diastolic left ventricular wall, mm | $1.46 \pm 0.13^a$ | $1.50 \pm 0.13^a$ | $1.45 \pm 0.11^a$ | 0.3256 |
| Diastolic left ventricular posterior wall, mm | $1.54 \pm 0.13^a$ | $1.67 \pm 0.16$ | $1.54 \pm 0.15^a$ | 0.0046 |
| Diastolic left ventricular inner diameter, mm | $6.06 \pm 0.42^a$ | $6.33 \pm 0.35^a$ | $6.37 \pm 0.72^a$ | 0.1082 |
| Systolic left ventricular inner diameter, mm | $3.32 \pm 0.54^a$ | $3.43 \pm 0.28^a$ | $3.82 \pm 1.01$ | 0.0445 |

(continued)

| Parameters | Midodrine (group II) | Atenolol (group III) | Control (group IV) | P-value |
|---|---|---|---|---|
| Left ventricular fractional shortening, % | 45.48 ± 6.25[a] | 45.70 ± 5.82[a] | 38.77 ± 8.59 | 0.0019 |
| Left ventricular ejection fraction, % | 81.55 ± 6.12[a] | 82.00 ± 5.31[a] | 73.87 ± 10.13 | 0.0007 |
| Left ventricular mass measured by ASE, g | 1.04 ± 0.06 | 1.10 ± 0.07[a] | 1.07 ± 0.12[a] | 0.0313 |
| Body weight, g | 238.24 ± 11.69[a] | 296.46 ± 16.73[b] | 236.20 ± 7.58[a] | 0.009 |
| Hear weight, g | 1.47 ± 0.16[a] | 1.47 ± 0.15[a] | 1.78 ± 0.35[a] | 0.062 |

**3-7: Effect of midodrine administration on adiponectin expression and fat profile**

The effect of long-term administration of midodrine on adiponectin expression and fat profile was investigated according to the method of Example 2-6.

In order to investigate how α1-AR stimulation activates AMPK, the level of adiponectin was measured. As seen from Table 2, the group II showed remarkably higher serum adiponectin level per the weight of visceral fat as compared to the group IV.

[Table 2]

| | Basal control (group I) | Midodrine (group II) | Atenolol (group III) | Control (group IV) | *P*-value |
|---|---|---|---|---|---|
| Adiponectin (ng/mL/g) | 74796.6 ± 13898.0[a] | 7585.8 ± 182.3[b] | 6136.5 ± 574.7[b,c] | 5455.8 ± 709.7[c] | < 0.001 |

As seen from FIG. 2E, the expression level of the phosphorylated AMPK protein in the liver was the highest in the group II similarly to other organs such as the aorta, skeletal muscle, fat, etc. and was relatively lower in the group IV. Also, as seen from FIG. 7, the expression level of cholesterol synthase HMG-CoA reductase protein (HMGCR), which is the downstream enzyme of AMPK, in the liver was the lowest in the groups II and III and was high in the control group IV.

The fat profile in blood is shown in Table 3. The level of total cholesterol, LDL cholesterol and HDL cholesterol was higher in the control groups (groups I and IV) than the drug-administered groups (groups II and IV), but there was no significant difference in the triglyceride level between the groups.

From these results, it can be seen that midodrine is effective in treating hyperlipidemia.

[Table 3]

| Lipids (SI/L) | Basal control (group I) | Midodrine (group II) | Atenolol (group III) | Control (group IV) | *P*-value |
|---|---|---|---|---|---|
| Total cholesterol | 1.69 ± 0.29 | 0.73 ± 0.15[a] | 1.01 ± 0.27[a] | 1.66 ± 0.17 | < 0.001 |
| LDL cholesterol | 0.43 ± 0.06 | 0.15 ± 0.04[a] | 0.15 ± 0.03[a] | 0.40 ± 0.03 | < 0.001 |
| HDL cholesterol | 0.81 ± 0.08 | 0.49 ± 0.11[a] | 0.52 ± 0.04[a] | 0.87 ± 0.15 | < 0.001 |
| Triglyceride | 0.83 ± 0.33[a] | 0.43 ± 0.12[a] | 0.77 ± 0.33[a] | 0.77 ± 0.31[a] | 0.092 |

**3-8: Effect of reducing fat content in adipocytes, body weight and abdominal fat**

When the preadipocytes (3T3-L1) were treated with midodrine, the fat content in adipocytes was decreased as seen from FIG. 8A. In addition, when a 50 μM/L GSK0660 (PPAR (peroxisome proliferator-activated receptor) β/δ inverse agonist) mixture (1:2(w/w)) was added, the effect of midodrine was decreased. In addition, when western blotting was conducted on the midodrine-treated adipocytes according to the method of Example 2-3, the expression of PPAR-δ, p-AMPK and PGC-1α proteins suppressing fat synthesis/accumulation was increased as shown in FIG. 8B.

When the body weight and the weight of abdominal fat (visceral fat) were measured for the midodrine-administered rat (II), atenolol-administered rat (III) and unadministered 8-week-old hypertensive control rat (IV) of Example 1-2, midodrine administration decreased both the body weight and abdominal fat as shown in FIG. 9.

From these results, it was confirmed that use of midodrine exhibits an effect of reducing body weight and

preventing heart failure in addition to lowering blood sugar.

In addition, it can be seen that $\alpha$1-AR stimulation by midodrine or a pharmaceutically acceptable salt thereof, which is an $\alpha$1-AR agonist, can provide a physiological effect exerted by exercise in the skeletal muscle and liver, improve left ventricular ejection fraction without accompanying cardiac hypertrophy or blood pressure increase, and change biochemical responses during the early hypertensive stage of spontaneously hypertensive rat. These are directly related with cardiac muscle contraction, cardiac exercise-mimetic effect and AMPK activation.

While the present disclosure has been described with respect to the specific embodiments, it will be apparent to those skilled in the art that various changes and modifications may be made without departing from the spirit and scope of the disclosure as defined in the following claims.

## Claims

1. A pharmaceutical composition for treating or preventing diabetes, comprising midodrine or a pharmaceutically acceptable salt thereof as an active ingredient.

2. The pharmaceutical composition for treating or preventing diabetes according to claim 1, wherein the midodrine or the pharmaceutically acceptable salt thereof induces AMPK activation.

3. The pharmaceutical composition for treating or preventing diabetes according to claim 1, wherein the midodrine or the pharmaceutically acceptable salt thereof induces the expression of PPAR-$\delta$ or PGC-1$\alpha$.

4. The pharmaceutical composition for treating or preventing diabetes according to claim 1, wherein the pharmaceutical composition further comprises insulin.

5. The pharmaceutical composition for treating or preventing diabetes according to claim 4, wherein the pharmaceutical composition comprises 20-40 parts by weight of midodrine or a pharmaceutically acceptable salt thereof and 60-80 parts by weight of insulin based on the total weight of the pharmaceutical composition.

6. A method for treating diabetes, comprising administering a pharmaceutically effective amount of midodrine or a pharmaceutically acceptable salt thereof to a subject.

7. A use of midodrine for preparation of a drug for treating or preventing diabetes.

8. A dietary supplement for reducing blood sugar, comprising midodrine.

9. A pharmaceutical composition for treating or preventing hyperlipidemia, comprising midodrine or a pharmaceutically acceptable salt thereof as an active ingredient.

10. The pharmaceutical composition for treating or preventing hyperlipidemia according to claim 8, wherein the midodrine or the pharmaceutically acceptable salt thereof induces AMPK activation.

11. The pharmaceutical composition for treating or preventing hyperlipidemia according to claim 8, wherein the midodrine or the pharmaceutically acceptable salt thereof induces the expression of PPAR-$\delta$ or PGC-1$\alpha$.

12. A method for treating hyperlipidemia, comprising administering a pharmaceutically effective amount of midodrine or a pharmaceutically acceptable salt thereof to a subject.

13. A use of midodrine for preparation of a drug for treating or preventing hyperlipidemia.

14. A dietary supplement for improving hyperlipidemia, which comprises midodrine.

FIG. 1

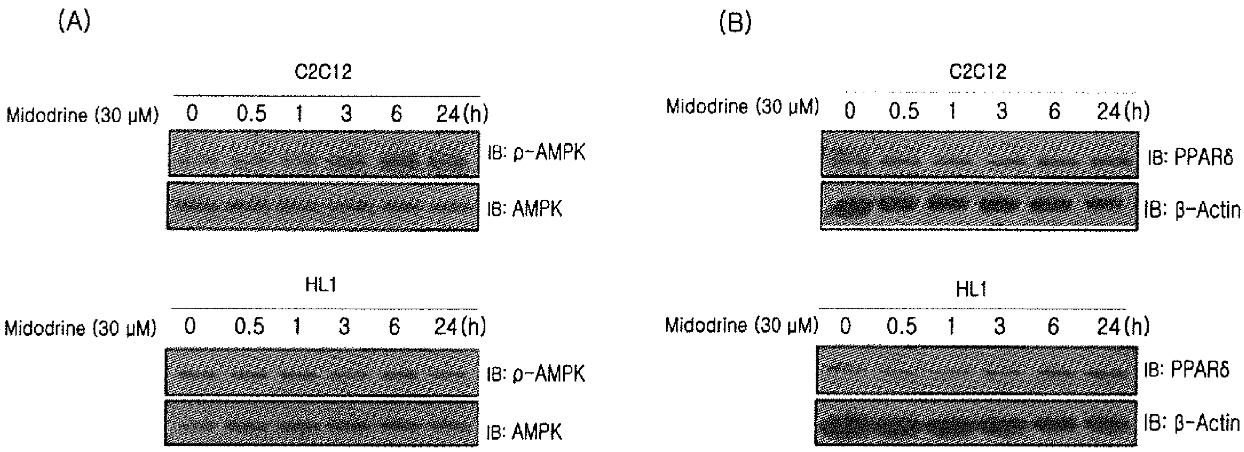
(A)
C2C12
Midodrine (30 μM) 0 0.5 1 3 6 24(h)
IB: p-AMPK
IB: AMPK
HL1
Midodrine (30 μM) 0 0.5 1 3 6 24(h)
IB: p-AMPK
IB: AMPK
(B)
C2C12
Midodrine (30 μM) 0 0.5 1 3 6 24(h)
IB: PPARδ
IB: β-Actin
HL1
Midodrine (30 μM) 0 0.5 1 3 6 24(h)
IB: PPARδ
IB: β-Actin

FIG. 2A

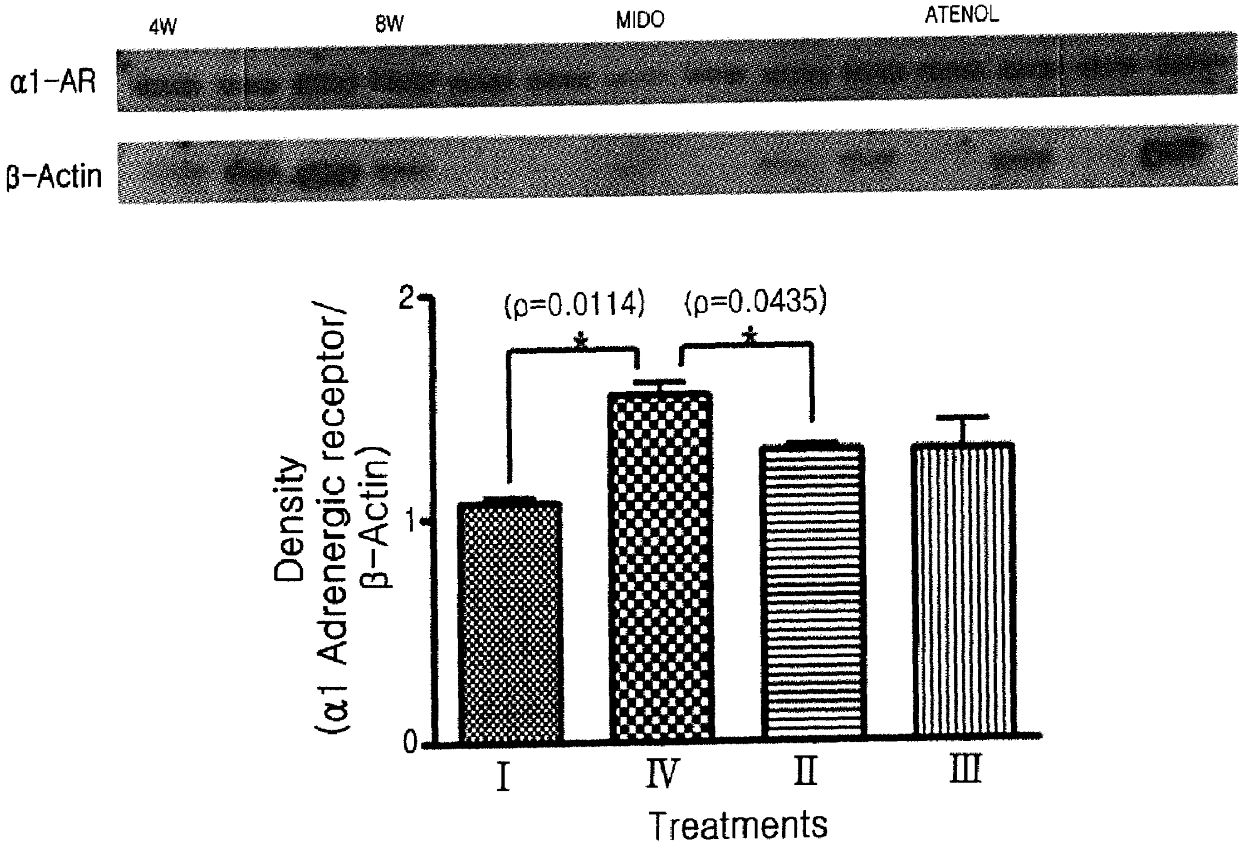
4W
8W
MIDO
ATENOL
α1-AR
β-Actin
Density
(α1 Adrenergic receptor/
β-Actin)
2
1
0
(p=0.0114)
(p=0.0435)
*
*
I
IV
II
III
Treatments

FIG. 2B

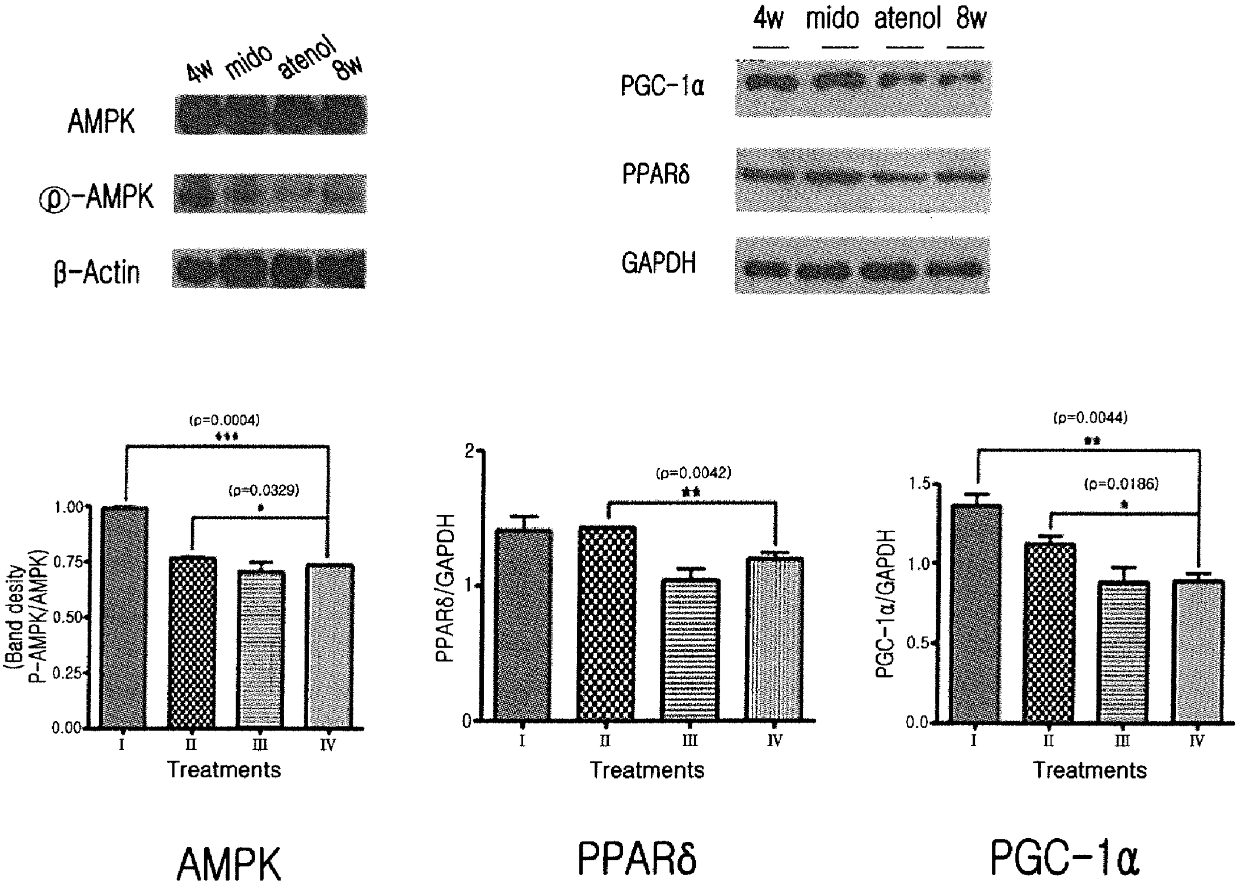
4w mido atenol 8w
AMPK
p-AMPK
β-Actin
PGC-1α
PPARδ
GAPDH
(p=0.0004)
***
(p=0.0329)
*
(Band desity P-AMPK/AMPK)
1.00
0.75
0.50
0.25
0.00
I II III IV
Treatments
(p=0.0042)
**
PPARδ/GAPDH
2
1
0
I II III IV
Treatments
(p=0.0044)
**
(p=0.0186)
*
PGC-1α/GAPDH
1.5
1.0
0.5
0.0
I II III IV
Treatments
AMPK
PPARδ
PGC-1α

FIG. 2C

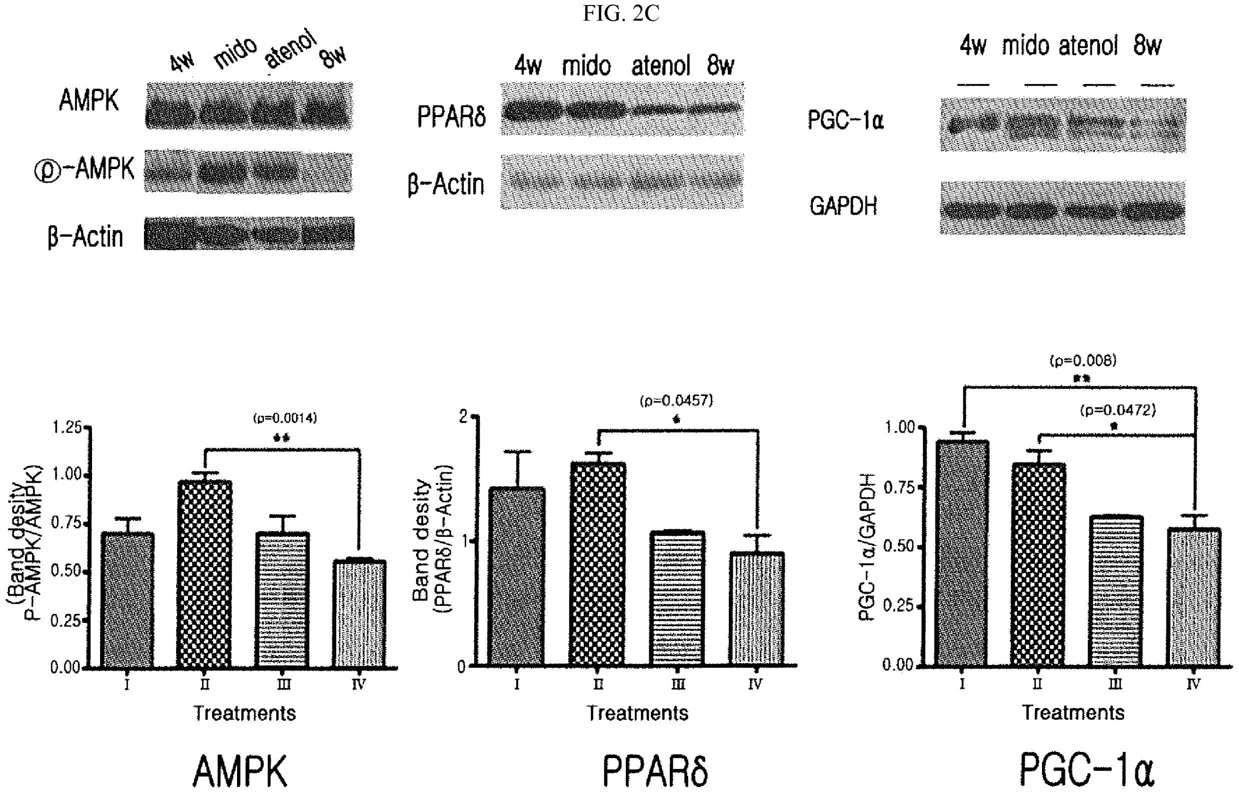
4w
mido
atenol
8w
AMPK
ⓟ-AMPK
β-Actin
PPARδ
β-Actin
PGC-1α
GAPDH
(p=0.0014)
**
Band desity (P-AMPK/AMPK)
1.25
1.00
0.75
0.50
0.25
0.00
I
II
III
IV
Treatments
AMPK
(p=0.0457)
*
Band desity (PPARδ/β-Actin)
2
1
0
Treatments
PPARδ
(p=0.008)
**
(p=0.0472)
*
PGC-1α/GAPDH
Treatments
PGC-1α

FIG. 2D

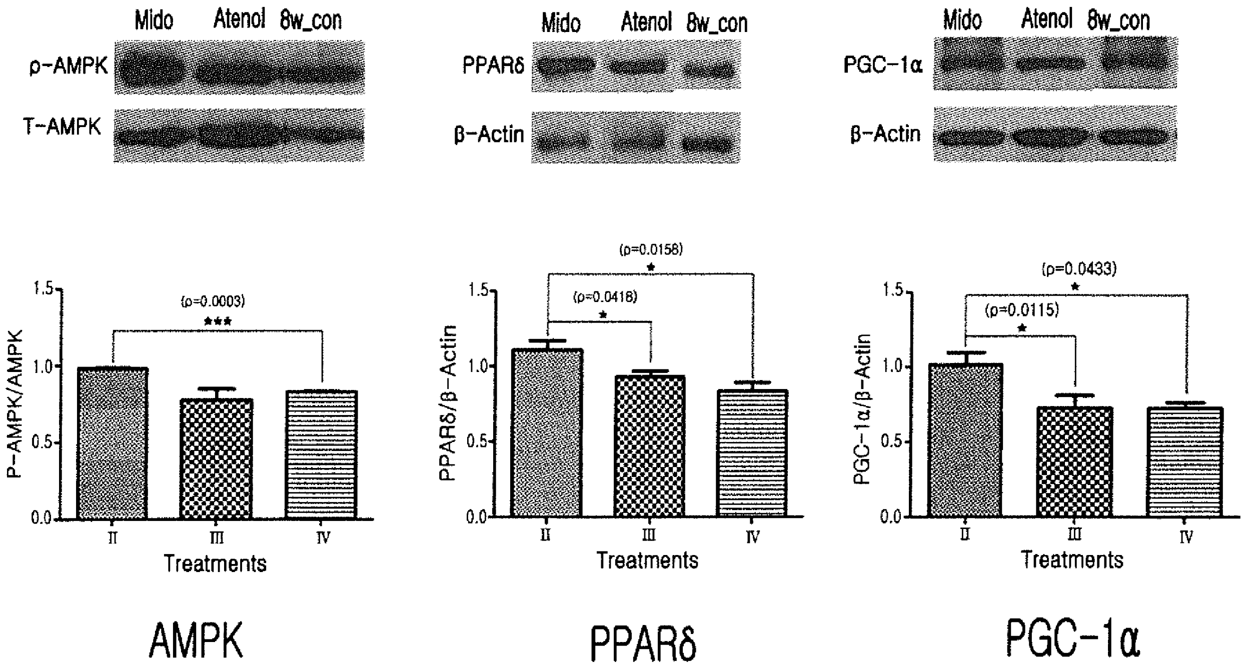
Mido Atenol 8w_con
p-AMPK
T-AMPK
PPARδ
β-Actin
PGC-1α
β-Actin
(p=0.0003)
***
P-AMPK/AMPK
(p=0.0158)
(p=0.0418)
PPARδ/β-Actin
(p=0.0433)
(p=0.0115)
PGC-1α/β-Actin
1.5
1.0
0.5
0.0
II III IV
Treatments
AMPK
PPARδ
PGC-1α

FIG. 2E

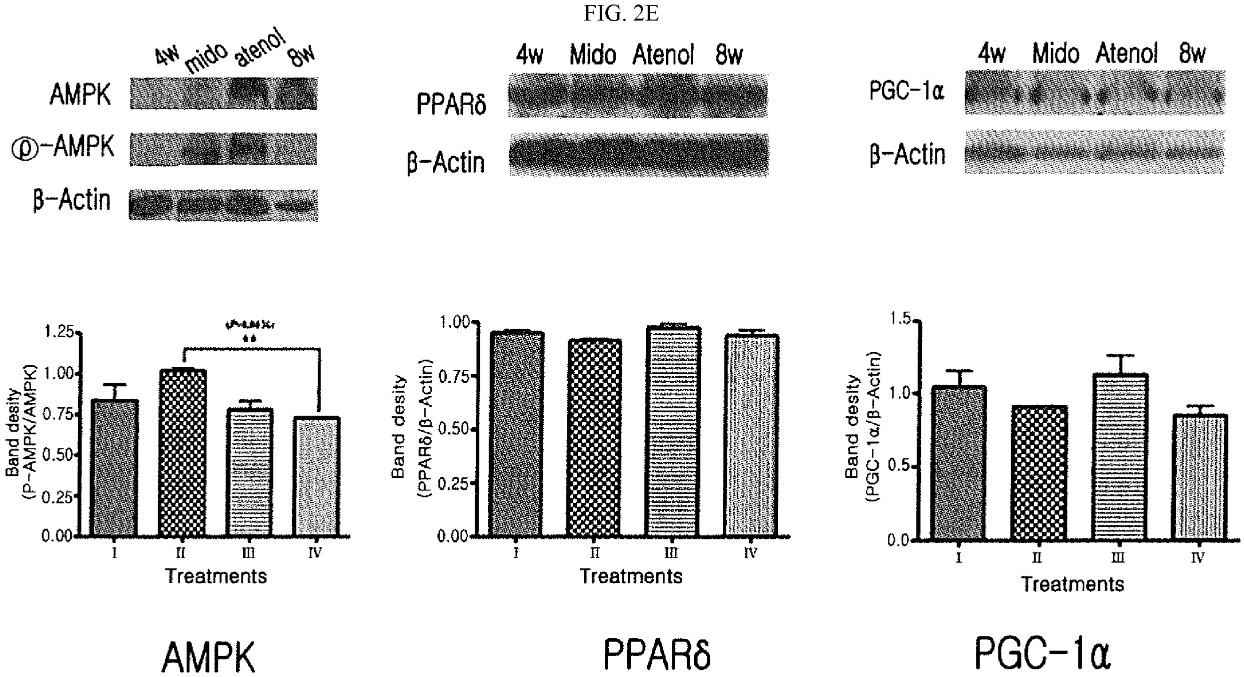
4w
mido
atenol
8w
AMPK
Ⓟ-AMPK
β-Actin
Band desity (P-AMPK/AMPK)
1.25
1.00
0.75
0.50
0.25
0.00
I
II
III
IV
Treatments
AMPK
4w Mido Atenol 8w
PPARδ
β-Actin
Band desity (PPARδ/β-Actin)
1.00
0.75
0.50
0.25
0.00
I
II
III
IV
Treatments
PPARδ
4w Mido Atenol 8w
PGC-1α
β-Actin
Band desity (PGC-1α/β-Actin)
1.5
1.0
0.5
0.0
I
II
III
IV
Treatments
PGC-1α

FIG. 3A

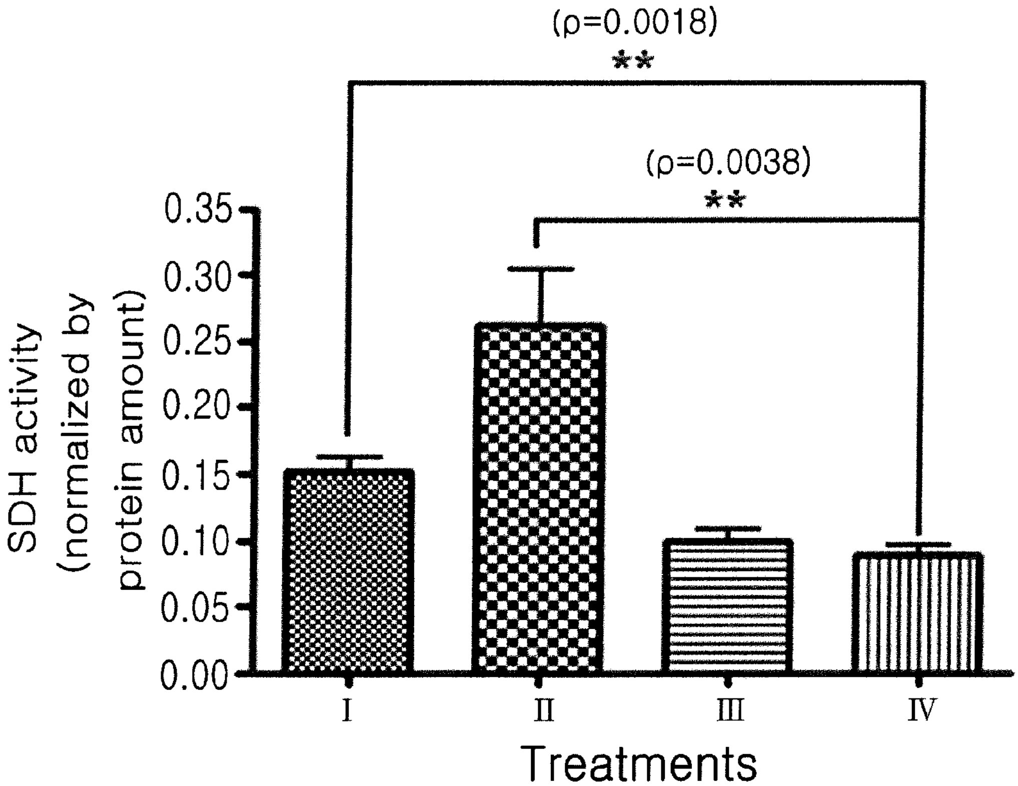
(ρ=0.0018)
**
(ρ=0.0038)
**
0.35
0.30
0.25
0.20
0.15
0.10
0.05
0.00
SDH activity
(normalized by
protein amount)
I
II
III
IV
Treatments

FIG. 3B

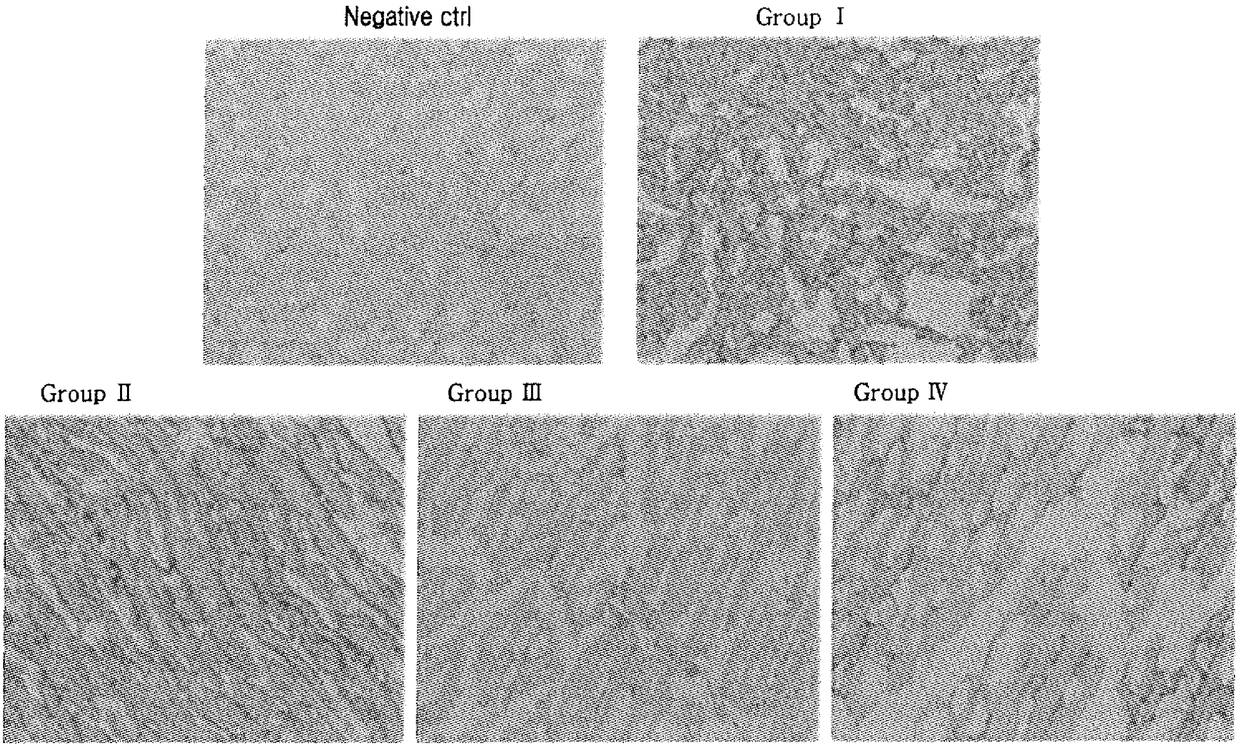

FIG. 4

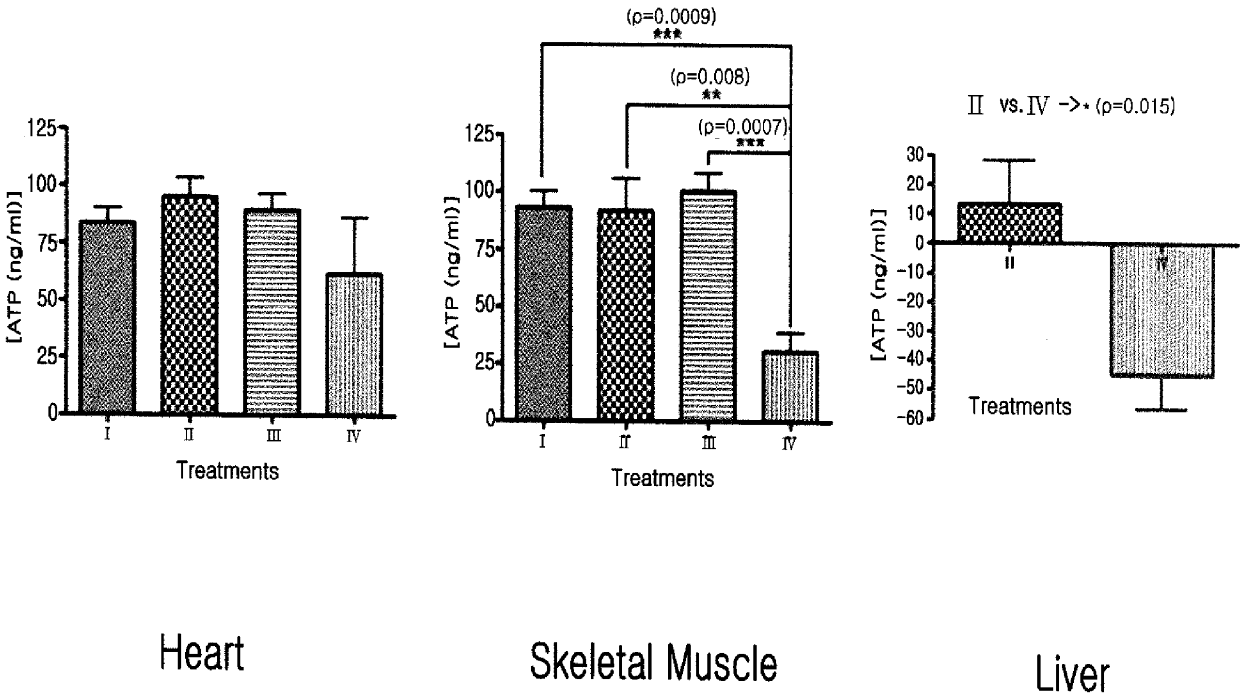
(p=0.0009)
***
(p=0.008)
**
(p=0.0007)
***
II vs. IV -> * (p=0.015)
[ATP (ng/ml)]
Treatments
Heart
Skeletal Muscle
Liver

FIG. 5

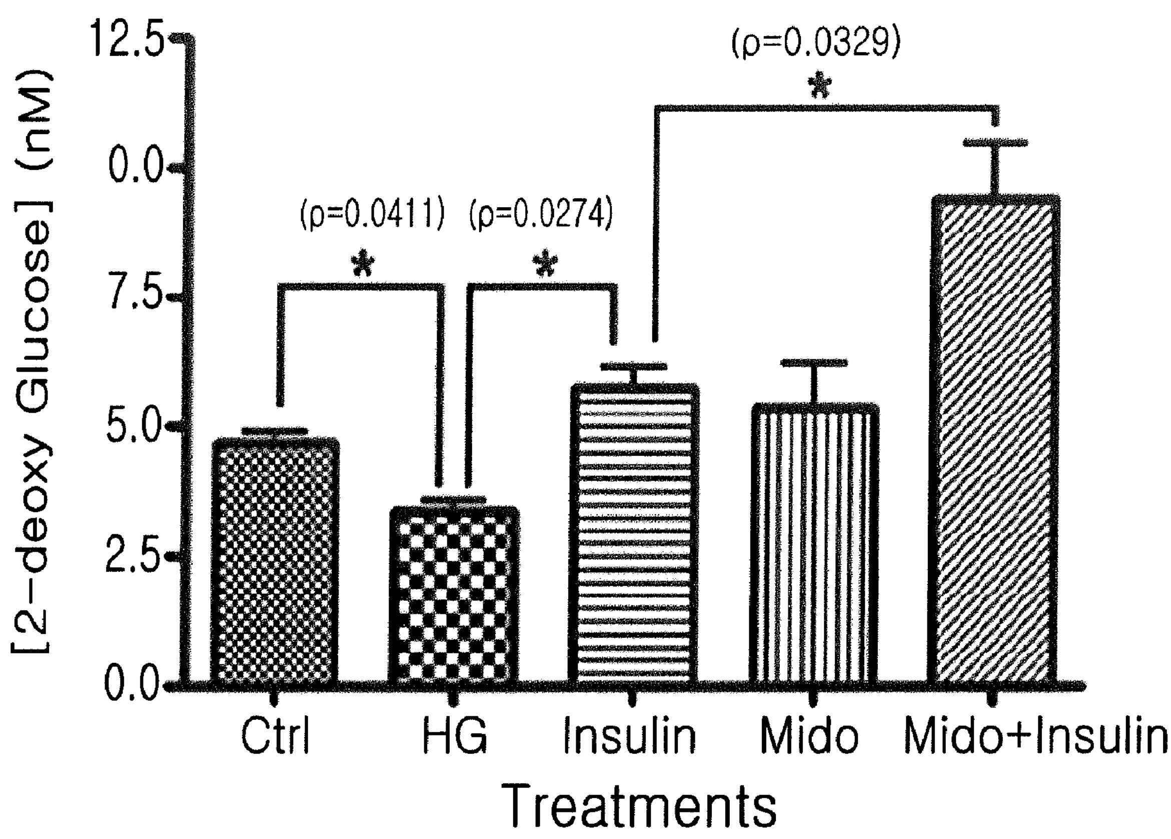
12.5
0.0
7.5
5.0
2.5
0.0
[2-deoxy Glucose] (nM)
(ρ=0.0411)
(ρ=0.0274)
(ρ=0.0329)
*
*
*
Ctrl
HG
Insulin
Mido
Mido+Insulin
Treatments

FIG. 6

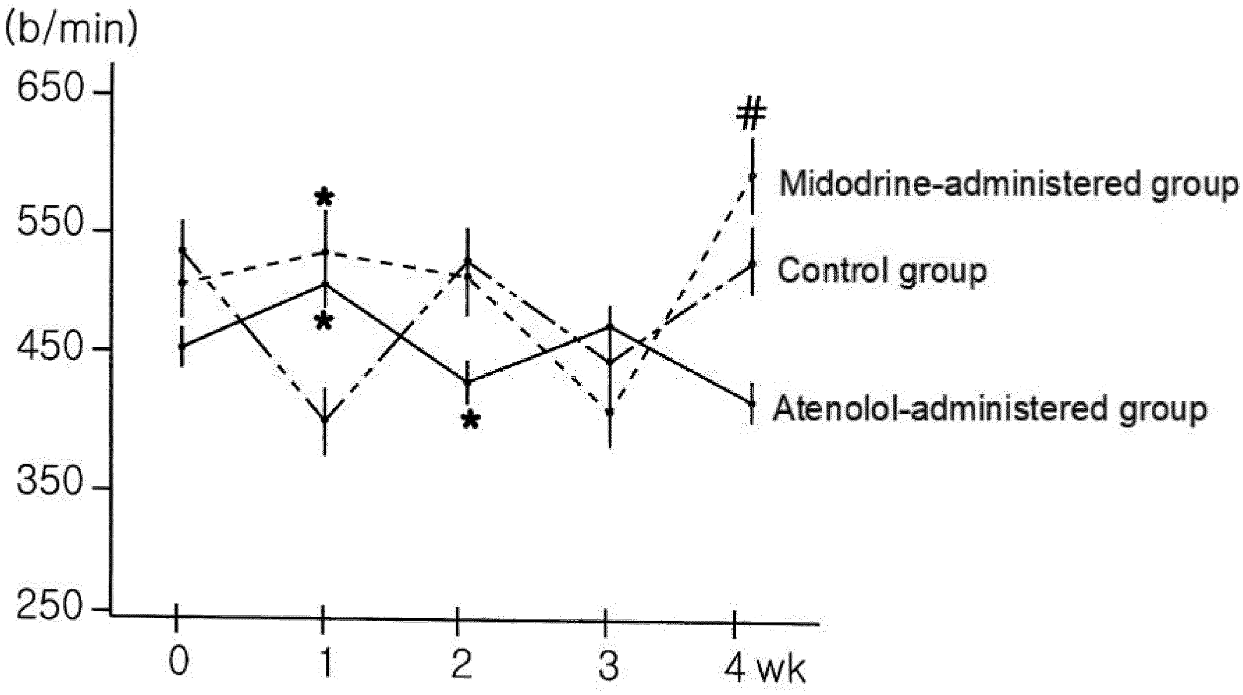
(b/min)
650
550
450
350
250
0
1
2
3
4 wk
#
*
*
*
Midodrine-administered group
Control group
Atenolol-administered group

FIG. 7

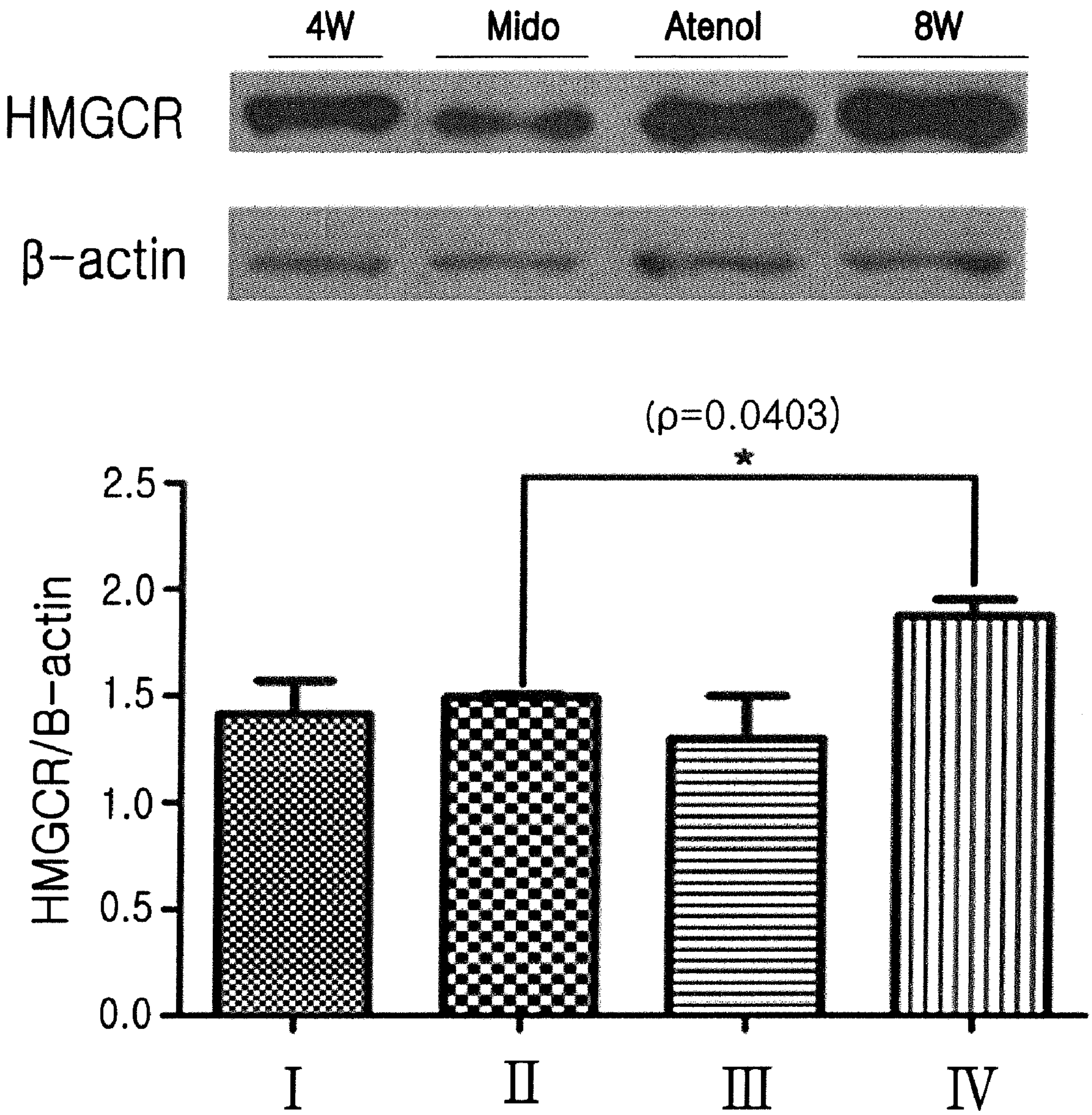
4W
Mido
Atenol
8W
HMGCR
β-actin
(p=0.0403)
*
2.5
2.0
1.5
1.0
0.5
0.0
HMGCR/B-actin
I
II
III
IV

FIG. 8A

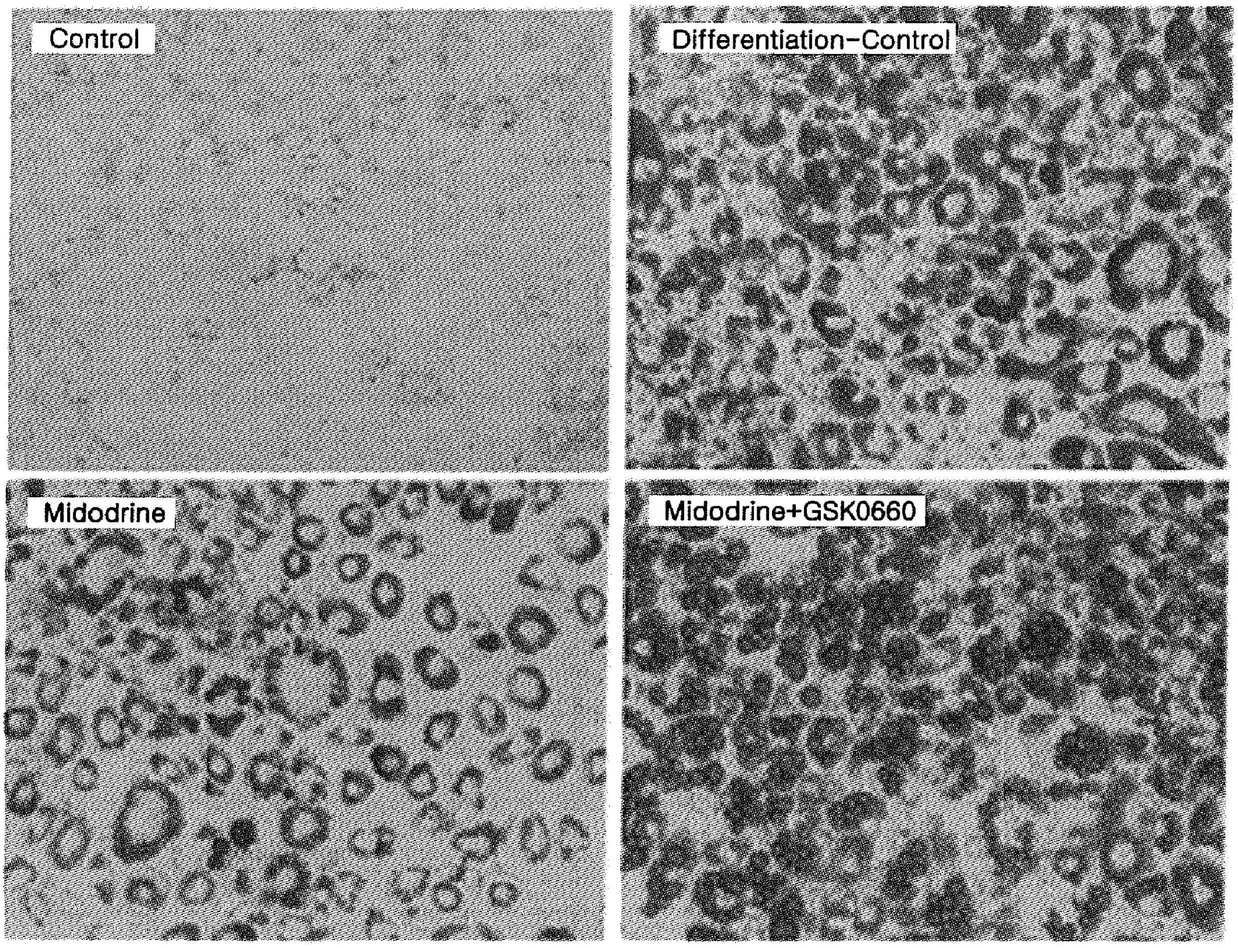
Control
Differentiation-Control
Midodrine
Midodrine+GSK0660

FIG. 8B

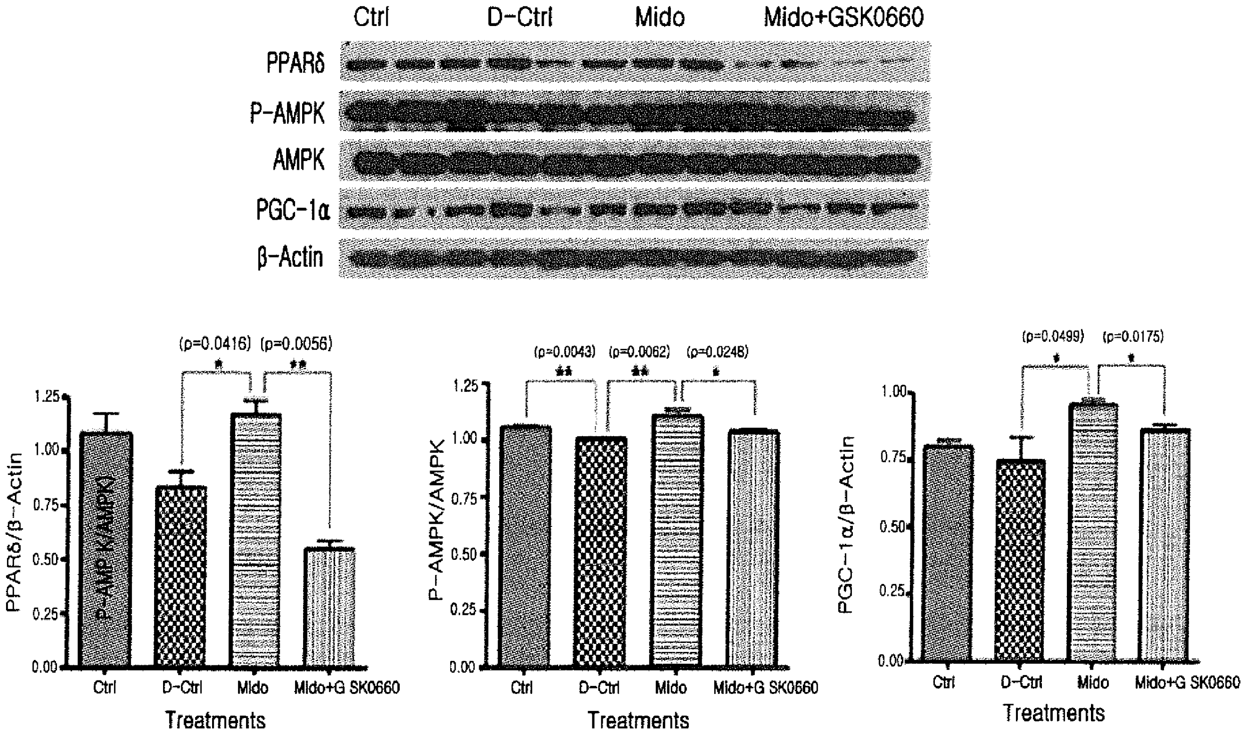
Ctrl
D-Ctrl
Mido
Mido+GSK0660
PPARδ
P-AMPK
AMPK
PGC-1α
β-Actin
(p=0.0416)
(p=0.0056)
PPARδ/β-Actin
P-AMPK/AMPK
Treatments
(p=0.0043)
(p=0.0062)
(p=0.0248)
(p=0.0499)
(p=0.0175)
PGC-1α/β-Actin
Mido+G SK0660

FIG. 9

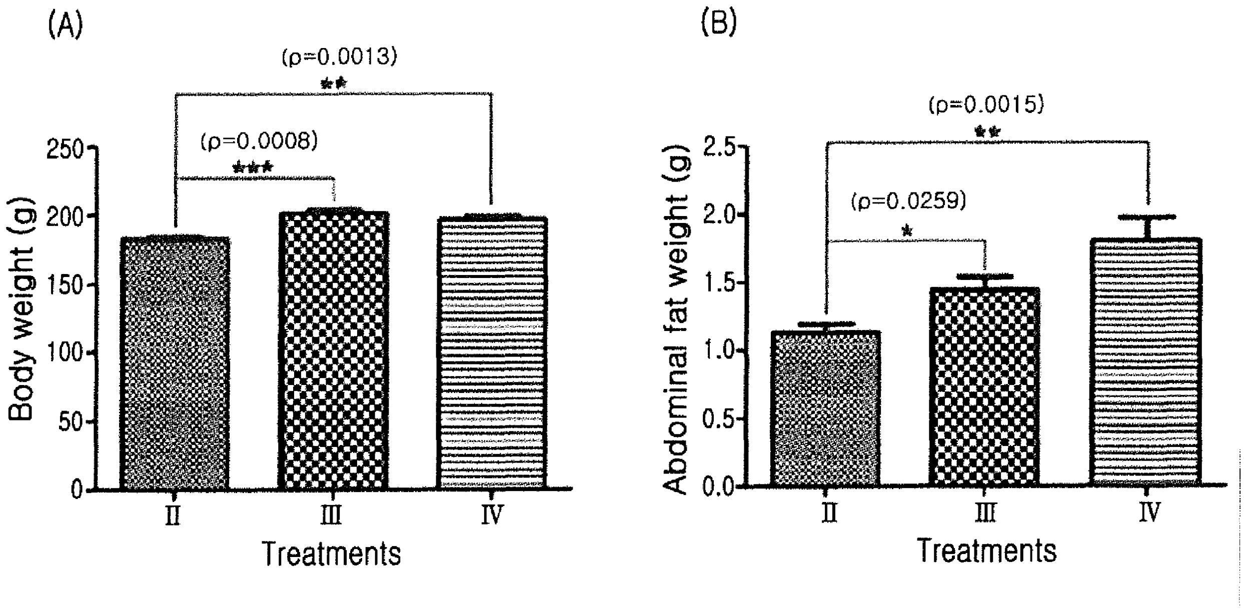
(A)
(p=0.0013)
**
(p=0.0008)
***
Body weight (g)
250
200
150
100
50
0
II
III
IV
Treatments
(B)
(p=0.0015)
**
(p=0.0259)
*
Abdominal fat weight (g)
2.5
2.0
1.5
1.0
0.5
0.0
II
III
IV
Treatments

**INTERNATIONAL SEARCH REPORT**

International application No.
**PCT/KR2017/011636**

A. CLASSIFICATION OF SUBJECT MATTER

***A61K 31/165(2006.01)i, A61K 9/14(2006.01)i, A61K 9/20(2006.01)i, A61K 9/48(2006.01)i, A61K 38/28(2006.01)i***

According to International Patent Classification (IPC) or to both national classification and IPC

B. FIELDS SEARCHED

Minimum documentation searched (classification system followed by classification symbols)
A61K 31/165; A61K 31/496; A61K 9/14; A61K 31/27; C07C 271/56; A61K 38/28; A61K 31/401; A61K 9/20; A61K 9/48

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal), STN Express(Registry, Caplus), Google & Keywords: midodrine, diabetes, hyperlipidemia, AMPK, PPAR-δ, PGC-1α

C. DOCUMENTS CONSIDERED TO BE RELEVANT

| Category* | Citation of document, with indication, where appropriate, of the relevant passages | Relevant to claim No. |
|---|---|---|
| X | WO 2008-100977 A2 (N.V. ORGANON) 21 August 2008<br>See paragraphs [0001], [0052], [00242], [00243]. | 1-5,7-11,13,14 |
| X | US 2004-0063719 A1 (ADAMS, M. A. et al.) 01 April 2004<br>See claims 1-3, 8. | 1-5,7-11,13,14 |
| A | PATHAK, A. et al., "Adverse Drug Reactions Related to Drugs Used in Orthostatic Hypotension: a Prospective and Systematic Pharmacovigilance Study in France", European Journal of Clinical Pharmacology, 2005 [Electronic publishing: 01 July 2005], vol. 61, nos. 5-6, pages 471-474<br>See the entire document. | 1-5,7-11,13,14 |
| PX | KR 10-1692680 B1 (KOREA UNIVERSITY RESEARCH AND BUSINESS FOUNDATION) 17 January 2017<br>See the entire document. | 1-5,7-11,13,14 |
| PX | KR 10-1749588 B1 (KOREA UNIVERSITY RESEARCH AND BUSINESS FOUNDATION) 21 June 2017<br>See the entire document. | 1-5,7-11,13,14 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 FEBRUARY 2018 (09.02.2018) | **09 FEBRUARY 2018 (09.02.2018)** |

Name and mailing address of the ISA/KR
Korean Intellectual Property Office
Government Complex-Daejeon, 189 Seonsa-ro, Daejeon 302-701, Republic of Korea
Facsimile No. +82-42-481-8578

Authorized officer

Telephone No.

Form PCT/ISA/210 (second sheet) (January 2015)

INTERNATIONAL SEARCH REPORT

International application No.
PCT/KR2017/011636

**Box No. II Observations where certain claims were found unsearchable (Continuation of item 2 of first sheet)**

This international search report has not been established in respect of certain claims under Article 17(2)(a) for the following reasons:

1. ☒ Claims Nos.: **6, 12**
because they relate to subject matter not required to be searched by this Authority, namely:
Claims 6 and 12 pertain to a method for treatment of the human body by therapy, and thus pertain to subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.
**PCT/KR2017/011636**

| Patent document cited in search report | Publication date | Patent family member | Publication date |
|---|---|---|---|
| WO 2008-100977 A2 | 21/08/2008 | WO 2008-100977 A3 | 18/12/2008 |
| US 2004-0063719 A1 | 01/04/2004 | AU 5403499 A | 21/03/2000 |
| | | CA 2340206 A1 | 09/03/2000 |
| | | EP 1235563 A2 | 04/09/2002 |
| | | US 2002-0035067 A1 | 21/03/2002 |
| | | US 2003-0008020 A1 | 09/01/2003 |
| | | US 2004-0234619 A1 | 25/11/2004 |
| | | US 6284763 B1 | 04/09/2001 |
| | | US 6458797 B1 | 01/10/2002 |
| | | US 6787553 B2 | 07/09/2004 |
| | | WO 00-12110 A2 | 09/03/2000 |
| | | WO 00-12110 A3 | 03/08/2000 |
| KR 10-1692680 B1 | 17/01/2017 | NONE | |
| KR 10-1749588 B1 | 21/06/2017 | NONE | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **VIHANG A. NARKAR et al.** AMPK and PPARδ Agonists Are Exercise Mimetics. *Cell,* 08 August 2008, vol. 134 (3), 405-415